(19) Europäisches Patentamt
European Patent Office
Office européen des brevets

(11) Veröffentlichungsnummer: **0 154 114**
**B1**

# (12) EUROPÄISCHE PATENTSCHRIFT

(45) Veröffentlichungstag der Patentschrift: **18.10.89**

(51) Int. Cl.⁴: **C 01 F 7/16**

(21) Anmeldenummer: **85100281.6**

(22) Anmeldetag: **12.01.85**

## (54) Neues Verfahren zur Herstellung von Magaldrate.

(30) Priorität: **08.03.84 DE 3408463**

(43) Veröffentlichungstag der Anmeldung:
**11.09.85 Patentblatt 85/37**

(45) Bekanntmachung des Hinweises auf die Patenterteilung:
**18.10.89 Patentblatt 89/42**

(84) Benannte Vertragsstaaten:
**AT BE CH DE FR GB IT LI LU NL SE**

(56) Entgegenhaltungen:
**DE-A-2 061 156**
**DE-C-963 182**
**US-A-3 418 087**

**CHEMICAL ABSTRACTS, Band 83, Nr. 10, 8. September 1975, Seite 371, Spalte 1, Zusammenfassungsnr. 84853e, Columbus, Ohio, US**
**USP-NF, 3. Ergänzung, S. 174**

(73) Patentinhaber: **Giulini Chemie GmbH, Giulinistrasse 2, D-6700 Ludwigshafen/Rhein (DE)**

(72) Erfinder: **Schanz, Klaus, Dr. Dipl.-Chem., In der Zeil 5, D-6701 Dannstadt-Schauernheim (DE)**

**EP 0 154 114 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der vorliegenden Erfindung ist ein neues Verfahren zur Herstellung von Magaldrate.

Gemäß United States Pharmacopoeia XX/1980, Third Supplement, USP-NF S. 174, wird mit Magaldrate ein Aluminium-Magnesium-Hydroxid-Sulfat der Formel

$$Al_5Mg_{10}(OH)_{31}(SO_4)_2 \cdot xH_2O$$

bezeichnet. Es kann sowohl im hydratisierten als auch im entwässerten Zustand hergestellt und eingesetzt werden und wird als chemische Kombination von Aluminiumhydroxid und Magnesiumhydroxid angesehen. Weiterhin enthält es nicht weniger als 29,0% und nicht mehr als 40,0% der äquivalenten Menge MgO und nicht weniger als 18% sowie nicht mehr als 26% der äquivalenten Menge $Al_2O_3$. Bezogen auf wasserfreies Magaldrate ergibt sich aus der Formel ein theoretischer Sulfat-Gehalt von ca. 17,5%.

Zur Charakterisierung von Magaldrate wird a.a.O. neben anderen auch der wasserlösliche Sulfatgehalt, der maximal bei 1,9% liegt, sowie das Röntgenbeugungsspektrum der USP-Magaldrate-Standardsubstanz im Bereich von 0,18 bis 2,20 nm herangezogen.

Magaldrate wird als antacide Wirksubstanz in bekannten Antacida eingesetzt. Die Eigenschaften dieser magaldratehaltigen Antacida, wie schnelle und lang andauernde Säurebindung, Alterungsstabilität, ausgeprägtes Adsorptionsvermögen gegenüber Pepsin, Gallensäure- und Lysolecithin werden aus der Zusammensetzung und der Struktur des Magaldrates erklärt.

Die Herstellung von Magaldrate, früher als Magnesiumaluminathydrat bezeichnet, ist seit langem bekannt. Gemäß der deutschen Patentanmeldung DE-C-963 182 wird dabei so vorgegangen, daß zu einer stark alkalischen Alkalialuminatlösung, die pro Mol $Al_2O_3$ drei bis fünf Mol $Na_2O$ oder ein anderes Alkalioxid enthält, bei einer 50 °C nicht übersteigenden Temperatur unter intensiver Durchmischung eine Magnesiumsalzlösung in einer solchen Menge zugegeben wird, daß das Verhältnis Aluminium zu Magnesium 1:0,9 bis 3 beträgt, der sich bildende Niederschlag von der Lösung abgetrennt, gewaschen und gegebenenfalls schonend getrocknet wird. Das angewandte Verhältnis von Aluminium zu Magnesium beträgt jedoch vorteilhafterweise 1:2.

Obwohl als wasserlösliches Magnesiumsalz bei dem vorstehenden Verfahren (Beispiele) ausschließlich Magnesiumsulfat verwendet wird, ist das Verfahren nicht auf dieses Magnesiumsalz beschränkt. Dies hat offensichtlich auch mit dazu geführt, daß weder in der deutschen Patentanmeldung noch in den früheren US-Pharmacopöen das Magaldrate als sulfathaltige Verbindung formuliert ist.

Wird gemäß der deutschen Patentanmeldung DE-C-963 182 gearbeitet und ein Atomverhältnis von Mg:Al wie 2 zu 1 eingehalten, erhält man das Magaldrate der angegebenen Formel, allerdings erst nach gründlichem Auswaschen des Filterkuchens. Der Filterkuchen kann nach Auswaschen mit Wasser zu einer Paste homogenisiert und unter Zusatz von Konservierungsstoffen, Geschmacksstoffen und anderen Hilfsmitteln zu einem flüssigen, antaciden Präparat verarbeitet werden. Alternativ kann der ausgewaschene Filterrückstand oder die Paste getrocknet werden und als antacider Wirkstoff bei der Herstellung von tabletten- bzw. pulverförmiger Antacida eingesetzt werden.

Es hat sich nunmehr überraschenderweise gezeigt, daß das zum Stand der Technik gehörende Verfahren durch ein neues, wesentlich besseres, ersetzbar ist. Fällung, Filtration, insbesondere Waschprozeß, sind bekanntlich bei dem vorstehenden Herstellungsprozeß langwierige und damit kapazitätsreduzierende Operationen, welche die Herstellungskosten im Vergleich zu denen anderer antacid wirksamer Stoffe, etwa Aluminiumhydroxidgel und Aluminiumphosphatgel, beträchtlich erhöhen. So wird beispielsweise für das Auswaschen von Aluminiumhydroxidgel, das nach dem Verfahren des deutschen Patentes 1 921 999 hergestellt worden ist, auf einer 100 $m^2$-Filterpresse eine Auswaschzeit von ca. 200 Min. benötigt, für Magaldrate hingegen, hergestellt nach der deutschen Patentanmeldung DE-C-963 182 (Stand der Technik), muß etwa mit dem vierfachen Zeitaufwand gerechnet werden, also mit ca. 800 Minuten.

Ein weiterer Nachteil des vorbekannten Verfahrens wird darin gesehen, daß bei der heute angewandten Filtrationstechnik mit Drehfiltern oder Filterpressen lediglich Filterkuchen-Konzentrationen von ca. 10–13 Gew.% Magaldrate erzielbar sind. Das hat zur Folge, daß sich bei der nachfolgenden Formulierung ausschließlich Endprodukte einstellen lassen, deren Magaldrate-Konzentration nur geringfügig unterhalb des angegebenen Konzentrationsbereiches liegt. Die derzeit im Handel befindlichen magaldratehaltigen flüssigen Antacida enthalten daher im Durchschnitt lediglich 9 Gew.%.

Aufgabe der vorliegenden Erfindung ist es daher, ein neues Verfahren zur Herstellung von Magaldrate zu finden, das gegenüber dem alten Verfahren sowohl arbeitstechnische als auch wirtschaftliche Vorteile aufweist und darüber hinaus neue galenische Anwendungsformen ermöglicht.

Die gestellte Aufgabe wird erfindungsgemäß in der Weise gelöst, daß aktives Aluminiumhydroxid mit mindestens einer wasserlöslichen sulfathaltigen Verbindung und aktivem Magnesiumoxid und/oder Magnesiumhydroxid in stöchiometrischen Mengen in Gegenwart von Wasser umgesetzt und die entstandene Magaldratepaste gegebenenfalls noch getrocknet wird. Die erhaltenen Magaldrate-Pasten enthalten keine Nebenprodukte. Filtrationen, Waschvorgänge und Rehomogenisierungsvorgänge sind nicht mehr erforderlich. Sie können unmittelbar zu flüssigen magaldratehaltigen Antacida weiterverarbeitet werden oder durch Trocknung, insbesondere Sprühtrocknung, in Magaldrate pulvis übergeführt werden. Außerdem hat

sich gezeigt, daß in Abhängigkeit von der Beschaffenheit der Ausgangsstoffe Konzentration und Viskosität der erzeugten Magaldrate-Pasten in einem weiten Bereich variiert werden können, was für die galenische Anwendung von besonderem Vorteil ist. Auch dies muß als überraschend bezeichnet werden und war aus dem Stand der Technik nicht herleitbar. Es können nunmehr Pasten mit bis zu 30 Gew.% Magaldrate-Gehalt erzeugt werden.

An dieser Stelle wird darauf hingewiesen, daß carbonatfreie Magaldrate nur dann erhalten werden, wenn der pH-Wert bei der Umsetzung von akt.Aluminiumhydroxid mit der sulfationenhaltigen Verbindung kleiner als 7 ist. Ist der pH-Wert gleich 7 oder größer als 7, enthalten die Produkte Kohlendioxid.

Aktives Aluminiumhydroxid ist bekannt. Es kann nach verschiedenen Verfahren hergestellt werden und ist ein feinteiliges, amorphes, in verdünnter Säure schnellösliches Aluminiumhydroxidgel. Es entsteht beispielsweise bei der Fällung aus Aluminiumsalzlösungen mittels Basen, insbesondere Alkalicarbonaten. Das nach der DE-PS 1 921 999 hergestellte Aluminiumhydroxidgel mit einem $CO_2$-Anteil von 12 bis 14% ist als Ausgangsprodukt hervorragend geeignet. Es kann sowohl in Pulver als auch in Pastenform eingesetzt werden. Sehr gut geeignet sind auch andere carbonathaltige Aluminiumhydroxidgel-Pasten, die der USP XX entsprechen. Aktive Aluminiumhydroxide können auch andere handelsübliche Aluminiumhydroxidgele sein, insbesondere solche, die aus Aluminiumchlorid hergestellt worden sind.

Die Verwendung von Aluminiumhydroxidgel pulvis, gemäß USP XX ist ebenfalls möglich. Beispielhaft werden hier die im Handel befindlichen Produkte Alugel® A 211 und Alugel® A 215 genannt.

Kombinationsprodukte, bestehend aus aktivem Aluminiumhydroxidgel gemäß USP XX und basischem Magnesiumcarbonat – sie sind im Handel als sogenanntes Aluminiumhydroxid-Magnesiumcarbonat-Co-Dried-Gel erhältlich – sind ebenfalls verwendbar.

Als wasserlösliche, sulfathaltige Verbindung kommen Aluminiumsulfat, Magnesiumsulfat und Schwefelsäure sowie Gemische dieser Verbindungen in Frage. Sie werden in pharmazeutischer Reinheit in solchen Mengen eingesetzt, daß die eingebrachte Sulfatmenge maximal 17,5%, bezogen auf die entstehende Magaldratemenge, beträgt. Wird Magnesiumsulfat allein eingesetzt, entstehen carbonathaltige Produkte.

Als aktives Magnesiumoxid wird in der vorliegenden Anmeldung ein Magnesiumoxid in pharmazeutischer Reinheit bezeichnet, das mit Wasser zu Magnesiumhydroxid reagiert. Zur Ermittlung der Aktivität kann die Jodzahl herangezogen werden, die angibt, wieviel mg Jod von 1 g Magnesiumoxid gebunden werden. Magnesiumoxide mit einer Jodzahl zwischen 20 und 100 sind als Reaktionspartner besonders geeignet.

Anstelle von Magnesiumoxid bzw. einer Mischung von Magnesiumoxid mit Magnesiumhydroxid kann auch Magnesiumhydroxid als Ausgangskomponente eingesetzt werden. Im zuletzt genannten Fall werden vorzugsweise Suspensionen (Pasten) von Magnesiumhydroxid eingesetzt. Magnesiumhydroxid-Suspensionen mit einem Gehalt von 30% $Mg(OH)_2$ sind im Handel mit unterschiedlichen Viskositäten erhältlich. Eine hochviskose Magnesiumhydroxid-Paste ist z.B. Gilumag® D 661 und eine niedrigviskose Gilumag® D 611.

Nach der bevorzugten Ausführungsform der Erfindung wird das aktive Aluminiumhydroxid als Suspension oder Paste vorgelegt. Unter Rühren werden stöchiometrische Mengen der in Wasser gelösten sulfathaltigen Verbindung, z.B. Aluminiumsulfat und/oder Schwefelsäure eingebracht. Nach Beendigung der hierbei zu beobachtenden Kohlendioxidentwicklung wird unter weiterem Rühren, gegebenenfalls auch Verdünnung mit Wasser, Magnesiumoxid zugegeben. Der Reaktionsablauf kann an der zu beobachtenden Temperaturerhöhung verfolgt werden. Nach einer Standzeit von 10 bis 24 Stunden ist die Paste zur Weiterverarbeitung geeignet, gegebenenfalls nach Vermahlung in einer Kolloidmühle.

Für die Berechnung der stöchiometrischen Mengen ist Basis das entstehende Magaldrate.

Die erfindungsgemäß hergestellte Magaldrate-Paste kann unmittelbar als Wirkstoff in pastenförmigen oder flüssigen Antacida verwendet werden. Sie kann selbstverständlich auch nach an sich bekannten Verfahren getrocknet werden, z.B. Sprühtrocknung und Walzentrocknung.

An dieser Stelle muß darauf hingewiesen werden, daß bei dem erfindungsgemäßen Verfahren nicht nur die Auswahl der Ausgangsstoffe kritisch ist – es bildet sich z.B. kein Magaldrate bei Verwendung von technischer Tonerde (Hydrargillit) – sondern auch die Reihenfolge der Reaktionsschritte kritisch ist. Durch kurze Vorversuche kann jedoch eine optimale Auswahl unter den genannten Reaktionskomponenten ohne Schwierigkeiten getroffen werden.

In der Regel wird man – wie bereits erwähnt – so vorgehen, daß man zu dem aktiven Aluminiumhydroxid zunächst die sulfathaltige Komponente gibt und dann das Magnesiumoxid. Verwendet man hier anstelle von Magnesiumoxid Magnesiumhydroxid, so entsteht kein brauchbares Magaldrate. Bei Verwendung von Magnesiumhydroxid allein erhält man ein brauchbares, allerdings carbonathaltiges Magaldrate dann, wenn das Magnesiumhydroxid vorgelegt wird, dann mit der sulfathaltigen Komponente und zum Schluß mit dem aktiven Aluminiumhydroxid versetzt wird.

Anhand der nachstehenden Beispiele wird der Gegenstand der Erfindung nunmehr noch näher erläutert:

Beispiel 1

In einem Becherglas, das mit einem Propellerrührer versehen ist, werden 261 g Aluminiumhydroxidgel-Paste, USP XX, mit einem $Al_2O_3$-Gehalt von 10,26% (ALUGEL® A 611) vorgelegt. Die Paste wird unter Rühren bei Raumtemperatur mit 250 ml Wasser verdünnt. Zu der verdünnten Aluminiumhydroxidgel-Paste werden 120 g Aluminiumsulfat-Lösung mit einer Dichte von 35°Bé, das entspricht einem $Al_2O_3$-Gehalt von ca. 8% und einem Sulfatgehalt von 22,6%, zugegeben. Die Reaktionsmischung wird noch so lange gerührt, bis die visuell zu beobachtende $CO_2$-Entwicklung weitgehend abgeschlossen ist. Das sind etwa 10 Minuten. Dann werden unter weiterem Rühren noch 310 ml Wasser zugegeben, anschließend 58,4 g leichtes Magnesiumoxid, USP XX, mit einer Jodzahl von 70. Während der Einarbeitung des Magnesiumoxids tritt eine deutliche Temperaturerhöhung ein. Nach beendeter Magnesiumoxidzugabe wird der Rührer gestoppt und der Ansatz 24 Stunden sich selbst überlassen. Das Reaktionsgemisch ist nun gut rührbar und wird zur Verbesserung der Konsistenz über eine Labor-Kolloidmühle gegeben.

Eine schonend abgetrocknete Suspensionsprobe wird einer röntgenographischen Untersuchung unterzogen. Für Vergleichszwecke wird dazu eine Röntgenaufnahme eines USP-Referenz-Standard-Magaldrates aufgenommen. Auswertung und Vergleich beider Röntgenaufnahmen zeigen, daß sich bei dem neuen Verfahren Magaldrate gebildet hat. Daß die Umsetzung praktisch 100%ig erfolgt ist, geht u.a. aus der Bestimmung des löslichen Sulfatgehaltes in der Suspension hervor. Der Wert von < 1,9% Sulfat (löslich) zeigt, daß der überwiegende Teil des eingebrachten Sulfats in chemisch gebundener Form vorliegt.

Die weitere analytische Untersuchung der Suspension ergibt einen Magnesiumoxidgehalt von 5,44%, einen $Al_2O_3$-Gehalt von 3,64, einen Natriumgehalt von 0,007% und einen pH-Wert von 9,1. Die Bestimmung des Magaldrate-Gehaltes in der Suspension kann rechnerisch auf der Basis der ermittelten MgO- und $Al_2O_3$-Gehalte, bzw. auf der Basis der eingebrachten Sulfatmenge berechnet werden. Eine weitere Möglichkeit besteht darin, die Suspension bei 105 °C einzudampfen und den Rückstand bis auf einen Trocknungsverlust von nicht mehr als 10% abzutrocknen. Eine mit dieser Bestimmung gut übereinstimmenden Methode, die im folgenden zu Grunde gelegt wird und die sich in der Praxis sehr gut bewährt hat, stellt die Gehaltsbestimmung durch Ermittlung der Säurekapazität dar.

Dazu wiegt man 10,0 g der entstandenen Magaldrate-Suspension in ein 250 ml Becherglas ein, fügt 30,0 ml 1 N Salzsäure zu und rührt, bis die Lösung klar ist. Dann wird mit 1 N Natronlauge die überschüssige Salzsäure bis pH 3 zurücktitriert. Aus dem sich ergebenen Säureverbrauch an 1 N Salzsäure läßt sich der Gehalt an Magaldrate berechnen. 1 g Magaldrate wasserfrei verbraucht 28,3 ml 1 N Salzsäure.

Die Konzentration der Suspension, in %, an wasserfreiem Magaldrate ergibt sich aus der verbrauchten 1 N Salzsäure, ausgedrückt in ml, multipliziert mit dem Faktor 100 und dividiert durch 28,3. Danach ergibt sich für die nach diesem Beispiel hergestellte Magaldrate-Suspension ein Magaldrate-Gehalt von 15,34%.

Beispiel 2

In einem Becherglas, das mit einem Propellerrührer versehen ist, werden 50,6 g Aluminiumhydroxidgel, pulvis, USP XX, Alugel®, Typ A 215, in 500 ml $H_2O$ susp. Zu dieser Suspension werden 120 g Aluminiumsulfat-Lösung mit einer Dichte von 35°Bé zugegeben. Nach einer Rührzeit von ca. 10 Minuten wird mit 270 ml Wasser verdünnt. Dann werden 58,4 g leichtes Magnesiumoxid, USP XX, mit einer Jodzahl von 90 eingerührt.

Nach einer Standzeit von 24 Stunden läßt sich die inzwischen abgesetzte Paste problemlos aufrühren. Zur Verbesserung der Sedimentationsstabilität und der Konsistenz wird die Suspension ebenfalls über eine Labor-Kolloidmühle gegeben. Das Produkt ist etwas dünnflüssiger als die im Beispiel 1 hergestellte Suspension und neigt zur stärkeren Sedimentation.

Die röntgenographische Auswertung zeigt wiederum, daß sich Magaldrate gebildet hat, wenngleich das Röntgenspektrum weniger gut ausgeprägt ist als bei dem Magaldrate Beispiel 1.

Der über die Säurekapazität bestimmte Magaldrate-Gehalt der Suspension beträgt 13,74%, der pH-Wert der Suspension liegt bei 9,8. Die Bestimmung erfolgt wie im Beispiel 1.

Beispiel 3

In einem Becherglas, das mit einem Rührwerk versehen ist, werden 85,9 g sprühgetrocknetes Aluminiumhydroxid-Magnesiumcarbonat-Co-Dried-Gel, (Typ C 220) in 300 ml Wasser suspendiert. Der $Al_2O_3$-Gehalt liegt bei ca. 42 Gew.% und der MgO-Gehalt bei ca. 7 Gew.%. Unter Rühren werden 28,1 g konzentrierte Schwefelsäure, verdünnt mit 300 ml Wasser, zugegeben. Nach 10-minütigem Rühren werden 235 ml Wasser zugesetzt und danach 51,9 g leichtes Magnesiumoxid, USP XX, mit einer Jodzahl von 30 eingerührt. Der Ansatz bleibt ebenfalls 24 Stunden sich selbst überlassen. Das dabei sich bildende Sediment ist sehr gut aufrührbar. Es entsteht eine dünnflüssige Suspension mit leicht sandigem Charakter. Zur Verbesserung der Sedimentationsstabilität und der Konsistenz des Produktes erfolgt eine Vermahlung über eine Labor-Kolloidmühle.

Die röntgenographische Auswertung zeigt eindeutig die Bildung von Magaldrate an. Die Konzentration, bestimmt über die Säurekapazität, liegt bei 14,77%, der pH-Wert der Suspension bei 8,0.

Beispiel 4

In einem Becherglas, das mit einem Rührwerk versehen ist, werden 357 g Aluminiumhydroxidgel-Paste, USP XX,* mit 10,2% $Al_2O_3$ vorgelegt und mit 150 ml Wasser verdünnt. Dazu werden 69,5 g Magnesiumsulfat-Heptahydrat, gelöst in 375 ml Wasser, gegeben. Unter weiterem Rühren werden 46,8 g leichtes Magnesiumoxid, USP XX, mit einer Jodzahl von 70 eingearbeitet. Der Ansatz bleibt 24 Stunden sich selbst überlassen und wird danach

* Alugel®, Typ A 611

aufgerührt und zur Verbesserung der Konsistenz des Produktes über eine Labor-Kolloidmühle gegeben.

Die röntgenographische Auswertung zeigt eindeutig die Bildung von Magaldrate an, die Konzentration, bestimmt über die Säurekapazität, liegt bei 15,52%. Der pH-Wert der Suspension wird mit 7,8 gemessen.

Die weitere Untersuchung des entstandenen Produktes zeigt einen wasserlöslichen Sulfatanteil von deutlich über 1,9%. Es zeigt sich weiter, daß der $CO_2$-Gehalt der Suspension deutlich gegenüber Suspensionen anderer Versuchsprodukte erhöht ist. Mit 1,6% ergibt sich ein $CO_2$-Gehalt der praktisch der gesamten $CO_2$-Menge entspricht, die mit dem eingesetzten Aluminiumhydroxidgel eingebracht wurde.

Beispiel 5

In einem Becherglas, das mit einem Rührwerk versehen ist, werden 357 g einer hochviskosen Aluminiumhydroxidgel-Paste, USP XX, Alugel®, Typ A 661 mit 10,2% $Al_2O_3$ vorgelegt und mit 150 ml Wasser verdünnt. Unter Rühren werden 28,1 g konzentrierte Schwefelsäure, verdünnt mit 300 ml Wasser zugegeben. Nach 10-minütigem Rühren werden 58,4 g leichtes Magnesiumoxid, USP XX, mit einer Jodzahl von 70 zugesetzt. Der Ansatz bleibt 24 Stunden sich selbst überlassen.

Das Reaktionsgemisch ist gut aufrührbar, wird jedoch zur Verbesserung der Konsistenz über eine Labor-Kolloidmühle gegeben. Die röntgenographische Auswertung zeigt eindeutig die Bildung von Magaldrate an, die Konzentration, bestimmt über die Säurekapazität, liegt bei 15,57%, der pH-Wert der Suspension bei 8,2.

Beispiel 6

In einem 150 Liter Reaktionsbehälter, der mit einem Rührwerk ausgelegt ist, werden 38,8 kg Aluminiumhydroxidgel-Paste, USP XX, Alugel® Typ A 611 mit 10,2% $Al_2O_3$ eingewogen. Unter Rühren wird zunächst mit 35,6 Liter Wasser weiter verdünnt. Anschließend werden unter Rühren 16,8 kg Aluminiumsulfat-Lösung mit 35°Bé zugemischt. Man rührt so lange, bis die visuell wahrnehmbare Gasentwicklung beendet ist. Danach werden 8,8 kg Magnesiumoxid, USP XX, mit einer Jodzahl von 70 zugegeben und durch weiteres Rühren in der Vorlage homogen verteilt. Unter Temperaturanstieg auf ca. 60 °C erfolgt ein deutlicher Viskositätsanstieg der Paste. Unter Rühren wird die Paste auf Zimmertemperatur abgekühlt. Es entstehen 100 kg Magaldrate-Paste mit einem Magaldrate-Gehalt von 23 Gew.%. Gemäß analytischer und röntgenographischer Auswertung ist Magaldrate entstanden.

Die bei diesem Ansatz erzielte Konsistenz des Produktes ist so, daß Hilfsstoffe noch direkt eingearbeitet werden können, jedoch liegt sie so hoch, daß eine ausreichende Transportstabilität gegen Sedimentation noch gewährleistet ist.

Das Beispiel zeigt, daß bei dem neuen Verfahren noch Magaldrate-Konzentrationen von 23 Gew.% erzielbar sind. Wie sich gezeigt hat, sind selbst Konzentrationen von 25 bis 30% erreichbar, wobei allerdings entsprechend höhere Viskositäten der Pasten in Kauf genommen werden müssen.

Beispiel 7

In einem Reaktionsbehälter aus Edelstahl, der mit einem Rührwerk versehen ist, werden 3 278 kg Aluminiumhydroxidgel-Paste, USP XX, Alugel® A 611 mit 11,37% $Al_2O_3$ vorgelegt und unter Rühren bei Normaltemperatur mit 5 500 Liter Wasser verdünnt. Unter fortgesetztem Rühren werden 1 566 kg Aluminiumsulfat-Lösung mit 35°Bé einfließen lassen. Es wird so lange gerührt, bis die Gasentwicklung weitgehend abgeschlossen ist, d.h. bis an der Oberfläche der Suspension visuell keine Gasentwicklung mehr zu beobachten ist. Danach werden 750 kg mittelschweres Magnesiumoxid, USP XX, mit einer Jodzahl von 20 eingetragen. Die Temperatur der Suspension erhöht sich auf ca. 60 °C. Unter Rühren läßt man die Suspension abkühlen, die Konsistenz der entstandenen Magaldrate-Suspension mit 19% Magaldrate ist derart, daß sie direkt einem Sprühtrockner oder einem anderen geeigneten Trocknungsaggregat durch Pumpen zugeführt werden kann.

Die Sprühtrocknung, bei einer Eintrittstemperatur von 330 °C und einer Austrittstemperatur von 115 °C durchgeführt, ergibt eine Ausbeute von 2 100 kg Magaldrate, pulvis. Die analytischen Ergebnisse des erhaltenen Sprühproduktes sind:

Beschreibung
Weißes, geruchloses, kristallines Pulver

Identifikation
a.) Magnesium – positiv
b.) Aluminium – positiv
c.) das Röntgenbeugungsspektrum entspricht dem des USP-Magaldrate-Referent-Standards im Bereich 0,18 bis 2,20 nm.

Löslichkeit
Unlöslich in Wasser und Alkohol, löslich in verdünnten Mineralsäuren

Magnesium (MgO)
31,18%

Aluminium ($Al_2O_3$)
21,03%

| | |
|---|---|
| Feuchte<br>bei 105 °C, 3 Stunden, 3,97% | Natrium (Na)<br>0,07% |
| Chlorid, wasserlöslich (Cl)<br>Spuren | Sulfat<br>wasserlöslich ($SO_4$)<br>< 1,9% |

Arsen (As)
< 8 ppm

Schwermetalle (als Pb)
< 60 ppm

E. coli
nicht nachweisbar

Das Produkt erfüllt die Anforderungen der USP XX.

Beispiel 8

In einem Becherglas, das mit einem Rührwerk versehen ist, werden 67,6 g Magnesiumhydroxid-Paste, USP XX, Gilumag® D 611 mit einem $Mg(OH)_2$-Gehalt von 30,5 Gew.% vorgelegt. In die Vorlage rührt man 28,25 g 8%ige Aluminiumsulfatlösung ein. Es entsteht eine hochviskose Paste, die mit 91,7 ml Wasser unter Rühren verdünnt wird. Die gebildete, gut durchmischbare Suspension wird mit 62,4 g einer Aluminiumhydroxidgel-Paste, USP XX, (A 611) mit einem $Al_2O_3$-Gehalt von 10,2% versetzt. Es entsteht eine Paste, die nach 24-stündiger Standzeit gut verrührbar ist. Das gebildete Magaldrate hat etwas sandigen Charakter und sedimentiert. Durch anschließende Vermahlung kann das Produkt homogenisiert werden.

Die Magaldrate-Konzentration der Paste liegt bei 15,5 Gew.% und der lösliche Sulfatgehalt im getrockneten Produkt bei 1,7%. Der MgO-Gehalt der Paste beträgt 5,70%, der $Al_2O_3$-Gehalt 3,60% und der gebundene $SO_4$-Gehalt 2,55%.

Die Fotocopien einer Röntgenaufnahme eines USP-Referenz-Standard-Magaldrates sowie des Magaldrates nach Beisp. 1 liegen bei.

**Patentansprüche**

1. Verfahren zur Herstellung von Magaldrate der Formel

$$Al_5Mg_{10}(OH)_{31}(SO_4)_2 \cdot xH_2O$$

dadurch gekennzeichnet, daß ein carbonathaltiges Aluminiumhydroxidgel mit einer wasserlöslichen sulfathaltigen Verbindung und einem Magnesiumoxid und/oder Magnesiumhydroxid mit einer Jodzahl zwischen 20 und 100 in stöchiometrischen Mengen in Gegenwart von Wasser umgesetzt und die entstandene Magaldrate-Paste gegebenenfalls noch getrocknet wird.

2. Verfahren nach Anspruch 1, dadurch gekennzeichnet, daß die Umsetzung bei einem pH-Wert kleiner als 7 durchgeführt wird.

3. Verfahren nach den Ansprüchen 1 und 2, dadurch gekennzeichnet, daß als wasserlösliche sulfationenhaltige Verbindung Aluminiumsulfat und/oder Schwefelsäure eingesetzt wird.

4. Verfahren nach Anspruch 1, dadurch gekennzeichnet, daß als wasserlösliche sulfationenhaltige Verbindung Magnesiumsulfat eingesetzt wird.

5. Verfahren nach den Ansprüchen 1 und 2, dadurch gekennzeichnet, daß als sulfationenhaltige Verbindung Magnesiumsulfat und Schwefelsäure eingesetzt werden.

6. Verfahren nach den Ansprüchen 1 bis 5, dadurch gekennzeichnet, daß als aktives Aluminiumhydroxid ein Aluminiumhydroxid-Magnesiumcarbonat-Gel eingesetzt wird.

7. Verfahren nach den Ansprüchen 1 und 3, dadurch gekennzeichnet, daß zu einer wäßrigen Dispersion von carbonathaltigem Aluminiumhydroxidgel unter intensiver Durchmischung Aluminiumsulfat-Lösung in stöchiometrischen Mengen gegeben wird, und daß nach abgeschlossener Kohlendioxidentwicklung stöchiometrische Mengen von Magnesiumoxid mit einer Jodzahl zwischen 20 und 100 in die Reaktionsmischung eingetragen werden, und daß nach einer Standzeit von 10 bis 24 Stunden die gebildete Magaldrate-Paste, gegebenenfalls nach Trocknung in Magaldrate, pulvis, der Weiterverarbeitung zugeführt wird.

**Claims**

1. Process for preparing magaldrates of formula

$$Al_5Mg_{10}(OH)_{31}(SO_4)_2 \cdot xH_2O$$

characterised in that a carbonate-containing aluminium hydroxide gel is reacted with a water-soluble sulphate-containing compound and a magnesium oxide and/or magnesium hydroxide with an iodine number of between 20 and 100 in stoichiometric quantities in the presence of water and the magaldrate paste formed is optionally also dried.

2. Process according to claim 1, characterised in that the reaction is carried out at a pH of less than 7.

3. Process according to claims 1 and 2, characterised in that aluminium sulphate and/or sulphuric acid is used as the water-soluble compound containing sulphate ions.

4. Process according to claim 1, characterised in that magnesium sulphate is used as the water-soluble compound containing sulphate ions.

5. Process according to claims 1 and 2, characterised in that magnesium sulphate and sulphuric acid are used as the compound containing sulphate ions.

6. Process according to claims 1 to 5, characterised in that an aluminium hydroxide – magnesium carbonate gel is used as the active aluminium hydroxide.

7. Process according to claims 1 and 3, characterised in that stoichiometric quantities of aluminium sulphate solution are added to an aqueous dispersion of carbonate-containing aluminium hydroxide gel with thorough mixing, and after the development of carbon dioxide has ceased, stoichiometric quantities of magnesium oxide with an iodine number of between 20 and 100 are added to the reaction mixture, and after a standing time of 10 to 24 hours the magaldrate paste formed is taken for further processing, optionally after being dried to form magaldrate powder.

**Revendications**

1. Procédé de préparation de magaldrate de formule

$$Al_5Mg_{10}(OH)_{31}(SO_4)_2 \cdot xH_2O$$

caractérisé en ce qu'on met en réaction en quantités stoechiométrique et en présence d'eau un gel d'hydroxyde d'aluminium contenant du carbonate avec un composé contenant du sulfate soluble dans l'eau et un oxyde et/ou un hydroxyde de magnésium dont l'indice d'iode est compris entre

20 et 100 et en ce qu'on sèche éventuellement encore la pâte de magaldrate formée.

2. Procédé selon la revendication 1, caractérisé en ce qu'on effectue la réaction à un pH inférieur à 7.

3. Procédé selon les revendications 1 et 2, caractérisé en ce qu'on met en œuvre, comme composé soluble dans l'eau contenant des ions sulfate, le sulfate d'aluminium et/ou l'acide sulfurique.

4. Procédé selon la revendication 1, caractérisé en ce qu'on met en œuvre, comme composé soluble dans l'eau contenant des ions sulfate, le sulfate de magnésium.

5. Procédé selon les revendications 1 et 2, caractérisé en ce qu'on met en œuvre, comme composé contenant des ions sulfate, le sulfate de magnésium et l'acide sulfurique.

6. Procédé selon les revendications 1 à 5, caractérisé en ce qu'on met en œuvre, comme hydroxyde d'aluminium actif un gel de carbonate de magnésiumhydroxyde d'aluminium.

7. Procédé selon les revendications 1 et 3, caractérisé en ce qu'une solution de sulfate d'aluminium est ajoutée en quantité stoechiométrique et en mélangeant intensément à une dispersion aqueuse de gel d'hydroxyde d'aluminium contenant du carbonate et en ce que après la fin du dégagement d'anhydride carbonique, des quantités stoechiométriques d'oxyde de magnésium d'indice d'iode compris entre 20 et 100 sont introduites dans le mélange réactionnel et en ce que, après une période de 10 à 24 heures, la pâte de magaldrate formée, éventuellement transformée par séchage en poudre de magaldrate, est amenée au traitement ultérieur.

USP - Standard - M

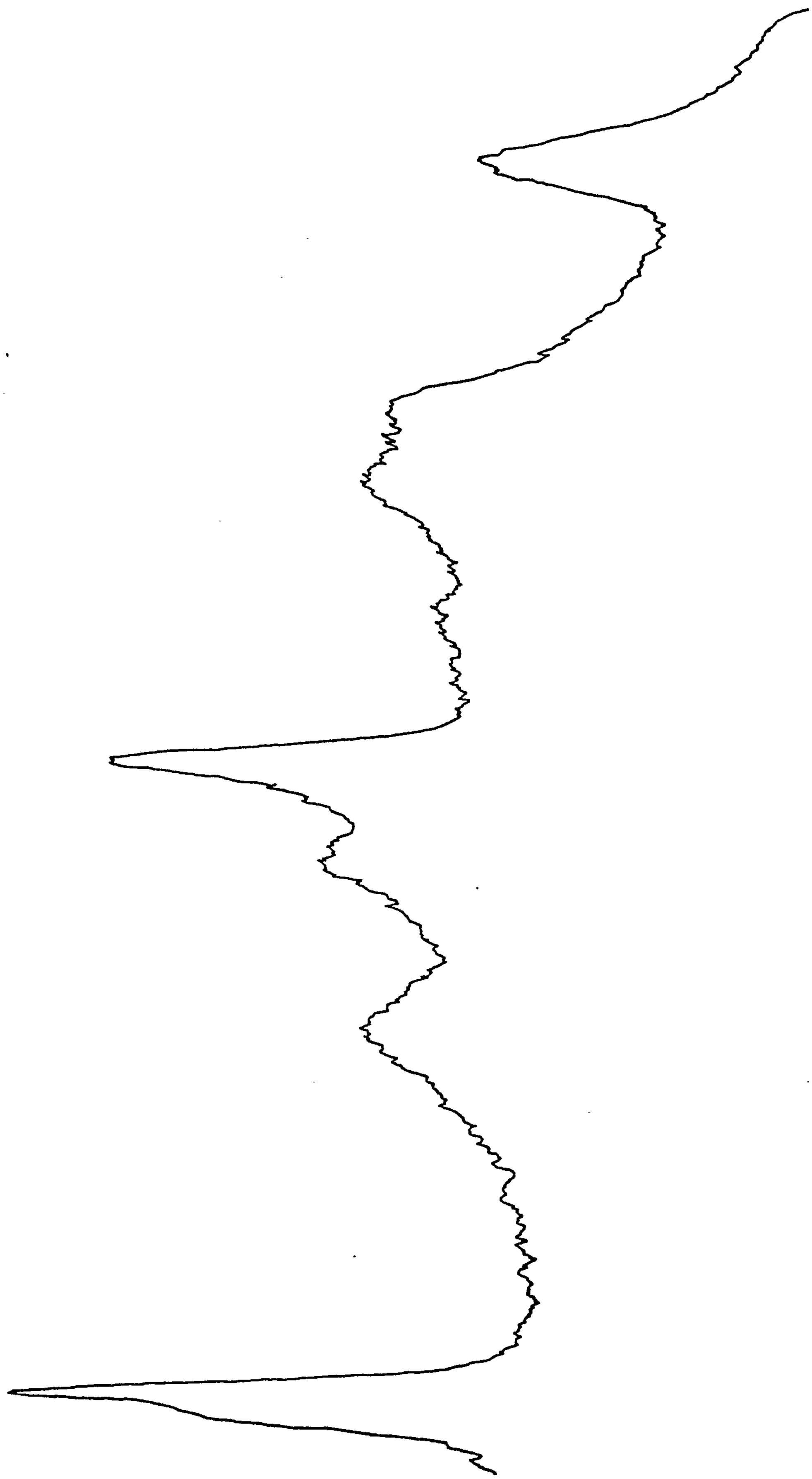

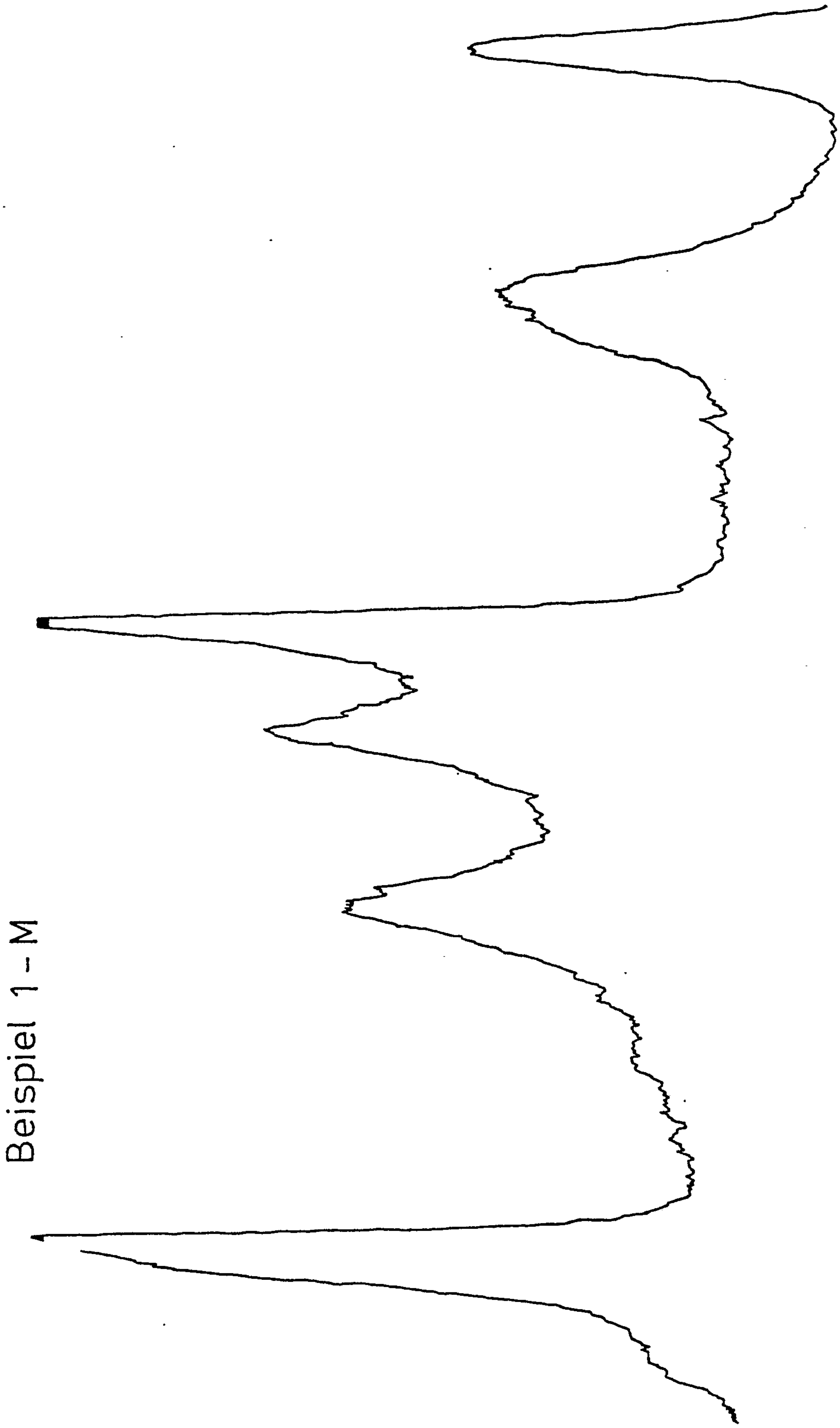

Beispiel 1 – M